(19) Europäisches Patentamt
European Patent Office
Office européen des brevets

(11) Publication number: 0 024 123
B1

(12) EUROPEAN PATENT SPECIFICATION

(45) Date of publication of patent specification: 16.03.83

(51) Int. Cl.³: C 07 D 498/04

(21) Application number: 80302476.9

(22) Date of filing: 22.07.80

# (54) Process for the preparation of derivatives of clavulanic acid.

(30) Priority: 11.08.79 GB 7928020
04.10.79 GB 7934526

(43) Date of publication of application:
25.02.81 Bulletin 81/8

(45) Publication of the grant of the patent:
16.03.83 Bulletin 83/11

(84) Designated Contracting States:
AT BE CH DE FR GB IT LI LU NL SE

(56) References cited:
DE - A - 2 657 048
DE - A - 2 754 763

(73) Proprietor: BEECHAM GROUP PLC
Beecham House Great West Road
Brentford Middlesex (GB)

(72) Inventor: Hunt, Eric
10 The Avenue Brockham
Betchworth Surrey (GB)

(74) Representative: Hesketh, Alan, Dr. et al,
European Patent Attorney Beecham
Pharmaceuticals Great Burgh Yew Tree Bottom
Road
Epsom, Surrey, KT18 5XQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 024 123 B1

Courier Press, Leamington Spa, England

# Process for the preparation of derivatives of clavulanic acid

This invention relates to a process for the preparation of clavulanic acid derivatives, and in particular to ether derivatives.

Belgian Patent Specification Number 864607 discloses an efficient process for the preparation of ethers of clavulanic acid including the methyl ether, by alkylation of an ester of calvulanic acid in the presence of silver ions. An alkaline earth metal oxide, such as calcium oxide is disclosed as usefully being present in that reaction.

It has now been found that if calcium sulphate or molecular sieve is present in the alkylation process of said Belgian patent, improved yields of product are obtained.

The present invention provides a process for the preparation of 9-O-methylclavulanic acid or a pharmaceutically acceptable salt or *in-vivo* hydrolysable ester thereof which process comprises the reaction of a cleavable ester of clavulanic acid with methyl iodide or methyl bromide in dry inert organic solvent in the presence of silver ions and either molecular sieve or calcium sulphate and subsequently:

i) converting any cleavable ester group which is not *in-vivo* hydrolysable into a free acid, a pharmaceutically acceptable salt or *in-vivo* hydrolysable ester group;

ii) optionally converting any cleavable ester group which is *in-vivo* hydrolysable into a free acid, a pharmaceutically acceptable salt or a different *in-vivo* hydrolysable ester group.

Most suitably methyl iodide is used as the alkylation reagent.

Typical inert organic solvents for this process include halogenated alkanes such as chloroform and methylene dichloride or ester solvents such as methyl acetate and ethyl acetate. A preferred solvent for this process is dichloromethane.

A particularly convenient source of silver ions is silver oxide.

Typical molecular sieves for use in this process are the alkali metal aluminium silicates such as sodium aluminium silicate, for example type 4A. Preferably sufficient molecular sieve or calcium sulphate is used to render the reaction mixture substantially anhydrous. Therefore the molecular sieve or calcium sulphate used in the process of this invention is preferably dry; that is to say substantially free of moisture.

Suitably for each equivalent of clavulanate ester 0.4—1.0 equivalents of silver oxide are used, more suitably 0.5—0.9 equivalents and preferably 0.6—0.8 equivalents. If calcium sulphate is used then suitably for each equivalent of clavulanate ester 1.0—8.0 equivalents of calcium sulphate are used, more suitably 2.0—6.0 equivalents and preferably 2.0—4.0 equivalents.

It is desirable to carry out the reaction in the dark to avoid formation of metallic silver.

One of the advantages of this reaction is that it can be performed at any convenient non-extreme temperature, for example, from 10° to 40°C. A particular advantage is that it is possible to carry out the process at ambient temperature (generally 20—30°C).

In general at room temperature the reaction is complete in 48—72 hours, but may be more rapid at higher temperatures.

The desired product may be obtained by filtering off the solids and evaporating the solvent. If desired this product may be purified by any convenient method such as chromatography or, for a solid ester, crystallisation.

The cleavable ester group employed in the process of this invention is one cleavable to yield the parent methyl ether or its salt. Such cleavage may be by chemical processes or by biochemical processes. Suitable chemical processes are mild base hydrolysis and catalytic hydrogenolysis. The biochemical process envisaged is enzymatic and may occur on isolated enzymes but will normally take place *in-vivo* after administration of the compound to the subject to be treated. Suitable cleavable esters of clavulanic acid and its ethers are disclosed in the aforementioned Belgian Patent Specification and in British Patent Specification Numbers 1508977 and 1508978.

Suitable ester groups for use in the process of this invention include those of the formula (I):

$$----CO_2R^1 \qquad (I)$$

wherein $R^1$ is a group $A^1$ or $CHA^2A^3$ wherein $A^{12}$ is an alkyl group of 1—8 carbon atoms, optionally substituted by halogen or a group of the formula $OA^4$, $OCOA^4$, $SA^4$, or $SO_2A^4$ wherein $A^4$ is a hydrocarbon group of up to 6 carbon atoms; $A^2$ is a hydrogen atom, an alkyl group of up to 4 carbon atoms or a phenyl group optionally substituted by halogen or by a group $A^5$ or $OA^5$ where $A^5$ is an alkyl group of up to 6 carbon atoms; and $A^3$ is a phenyl group optionally substituted by halogen or by a group $A^5$ or $OA^5$ where $A^5$ is an alkyl group; or $A^1$ is an alkenyl group of 2—8 carbon atoms, for example allyl.

Particularly suitable *in-vivo* hydrolysable esters include acetoxymethyl, α-acetoxyethyl, pivaloyloxymethyl, phthalidyl, ethoxycarbonyloxymethyl and ethoxycarbonyloxyethyl.

Preferred esters are those cleavable by mild base and include the methyl, ethyl, methoxymethyl and ethoxymethyl esters.

A particularly suitable ester for use in the process of this invention is the methoxymethyl ester.

A preferred ester for use in the process of this invention is the methyl ester.

A further suitable ester for use in the process of this invention is the benzyl ester.

From the foregoing it will be realised that in a preferred aspect this invention provides a process for the preparation of 9-O-methylclavulanic acid which process comprises the reaction of methyl clavulanate with methyl iodide in a dry inert organic solvent in the presence of silver oxide and molecular sieve or dry calcium sulphate, and hydrolysing the methyl ester group.

The starting materials for the process of this invention may be prepared by known methods for example as disclosed in British Patents 1508977 and 1508978. In particular methyl clavulanate may conveniently be prepared by the reaction of potassium clavulanate with methyl iodide in acetone.

In any of the above processes the drying agent may be a mixture of molecular sieve and calcium sulphate.

The following Examples illustrate the invention:

Example 1

Methyl 9-O-methyl clavulanate

i) Methyl clavulanate

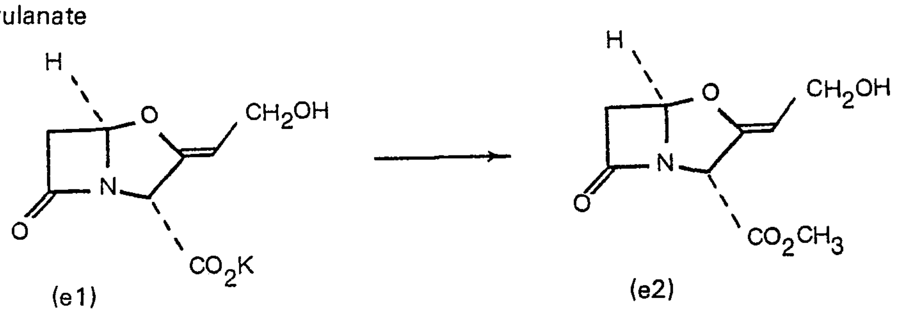

(e1) (e2)

Potassium clavulanate (e1) (1.2 g) was suspended in acetone (25 ml) and methyl iodide (1 ml) was added. The mixture was stirred at room temperature with the exclusion of moisture. After 1.5 hours methyl iodide (2 ml) was added and stirring continued for a further 22.5 hours. The reaction mixture was then heated to 50°C methyl iodide (2 ml) added and stirring continued for 48 hours. The mixture was diluted with ethyl acetate (50 ml), filtered and the solvent removed by evaporation. The residue was dissolved in ethyl acetate/petrol (1/1, 100 ml) and the solution stirred with charcoal and filtered. The solvent was removed by evaporation to yield methyl clavulanate (e2) (0.91 g) as a yellow gum.

ii) Methyl 9-O-methyl clavulanate

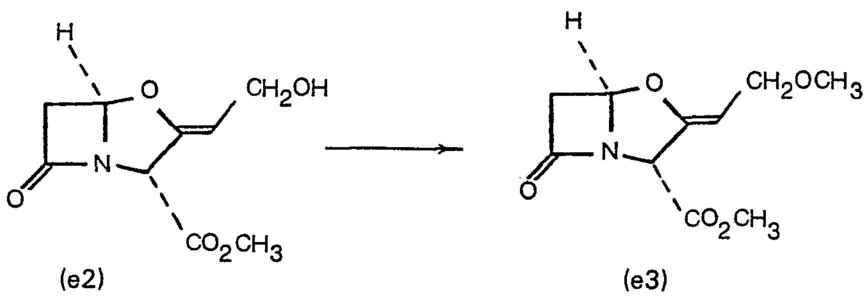

(e2) (e3)

Methyl clavulanate (e2) (430 mg) was dissolved in dry methylene dichloride (10 ml) containing methyl iodide (1 ml). Activated 4A molecular sieve powder (1.5 g) and silver oxide (0.7 g) were added and the mixture was stirred at room temperature in the dark with the exclusion of moisture. After 64 hours (when t.l.c. using 1/1 ethyl acetate/petrol showed no starting material remaining) the mixture was filtered and the solid washed with methylene dichloride (3 x 10 ml). The combined filtrate and washings were evaporated to yield methyl 9-O-methyl clavulanate (e3) as a pale yellow gum. This was purified by column chromatography on silica gel eluting with ethyl acetate/petrol (1:7 going to 1:2) to yield pure methyl 9-O-methyl clavulanate (e3) (250 mg) as a colourless gum.

## Example 2

Methyl 9-O-methyl clavulanate

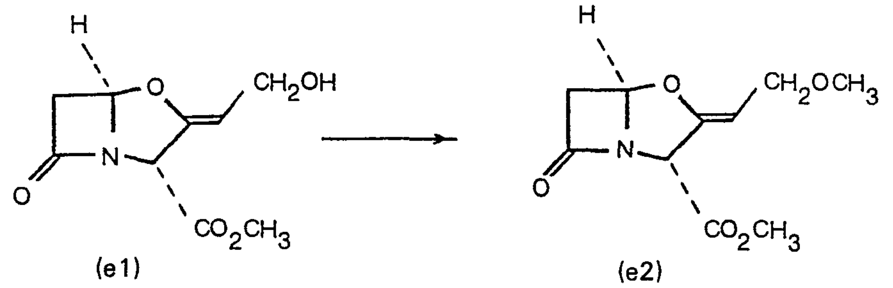

(e1) (e2)

Silver oxide (1.0 g) and calcium sulphate powder (2.0 g, "Drierite") were suspended in a solution of methyl clavulanate (e1) (850 mg) in dry methylene dichloride (10 ml). Methyl iodide (1.2 ml) was added to the mixture which was then stirred at room temperature in the dark for 48 hours. The mixture was filtered and the solid washed with dry methylene dichloride (3 x 10 ml). The solvent was removed from filtrate by evaporation and the resulting gum chromatographed on silica gel eluting with ethyl acetate/petrol (1:7 going to 1:2). The methyl 9-O-methyl clavulanate (e2) (570 mg) was obtained as colourless gum.

## Example 3

Calcium 9-O-methyl clavulanate

i) Benzyl 9-O-methyl clavulanate

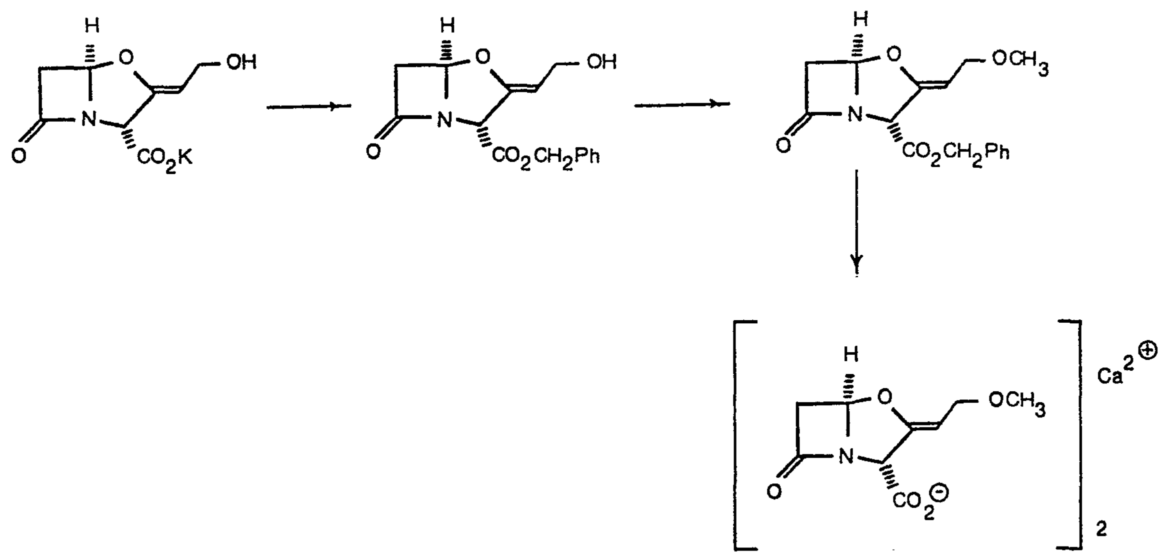

Potassium clavulanate (2.5 g) was suspended in a mixture of acetone (5 ml) and benzyl bromide (1.2 ml) and the mixture was stirred at 65°C (bath temperature) for 70 hours. The mixture was cooled, diluted with ethyl acetate (50 ml), and filtered. The solvent was evaporated from the filtrate and the resulting gum was dissolved in ethyl acetate (50 ml). Petroleum ether (b.p. 60—80°) (50 ml) was added to the solution which was then stirred with charcoal and filtered. The solvent was removed to yield benzyl clavulanate as a yellow gum (2.46 g).

The benzyl clavulanate (2.46 g) was dissolved in methylene dichloride (25 ml) and methyl iodide (1.5 ml) was added to the solution, followed by silver oxide (2.3 g) and anhydrous calcium sulphate powder (4.5 g). The mixture was stirred at room temperature in the dark for 44 hours. The mixture was diluted with ethyl acetate (50 ml) and filtered. The solvent was removed from the filtrate and the resulting gum was chromatographed on silica gel using ethyl acetate/petroleum ether (b.p. 60—80°) (1:7 going to 1:4). Benzyl 9-O-methyl clavulanate was thus obtained as an almost colourless gum (0.97 g).

ii) Calcium 9-O-methyl clavulanate

The above benzyl ester was converted into 9-O-methylclavulanic acid by hydrogenolysis over 10% palladium on charcoal in tetrahydrofuran at room temperature and atmospheric pressure. The acid was neutralised using calcium oxide and the resulting salt was isolated by evaporation of the solvent. Calcium 9-O-methyl clavulanate was thus obtained as an orange coloured powder (0.62 g).

## Example 4

Benzyl 9-O-methyl clavulanate

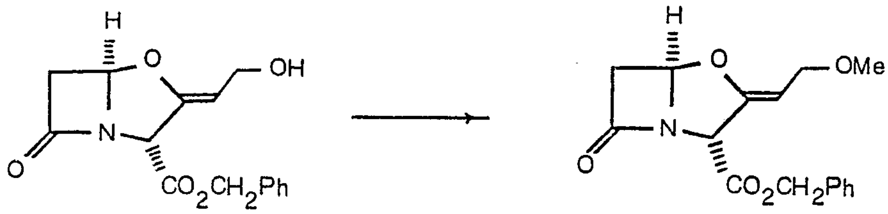

Benzyl clavulanate (9.28 g) and methyl iodide (10 ml) were dissolved in methylene dichloride (100 ml). Silver oxide (8 g) and anhydrous calcium sulphate powder (16 g) were added and the mixture was stirred at room temperature in the dark for 48 hours. The mixture was filtered, the solvent was removed from filtrate, and the resulting gum chromatographed on silica gel using ethyl acetate/petroleum ether (b.p. 60—80°). Benzyl 9-O-methylclavulanate was thus obtained as a pale yellow gum (7.19 g).

## Example 5

Methoxymethyl 9-O-methylclavulanate

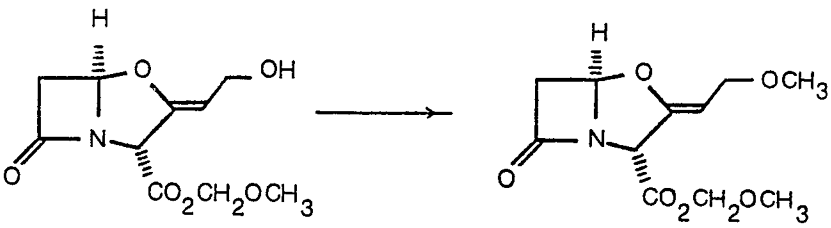

Methoxymethyl clavulanate (1.82 g) in methylene dichloride (10 ml) was treated with methyl iodide (1 ml), and anhydrous calcium sulphate powder (3.5 g). The mixture was stirred at room temperature in the dark for 48 hours. The mixture was filtered, the solvent was evaporated from the filtrate, and the resulting residue was chromatographed on silica gel using ethyl acetate/petroleum ether (b.p. 60—80°). Methoxymethyl 9-O-methylclavulanate was thus obtained as pale yellow crystals (600 mg), m.p. 50—52°.

**Claims**

1. A process for the preparation of 9-O-methylclavulanic acid or a pharmaceutically acceptable salt or *in-vivo* hydrolysable ester thereof which process comprises the reaction of a cleavable ester of clavulanic acid with methyl iodide or methyl bromide in dry inert organic solvent in the presence of silver ions, and subsequently:

i) converting any cleavable ester group which is not *in-vivo* hydrolysable into a free acid, a pharmaceutically acceptable salt or *in-vivo* hydrolysable ester group;

ii) optionally converting any cleavable ester group which is *in-vivo* hydrolysable into a free acid, a pharmaceutically acceptable salt or a different *in-vivo* hydrolysable ester group; characterised in that it is conducted in the presence of molecular sieve or calcium sulphate.

2. A process as claimed in claim 1 wherein the solvent is dichloromethane.

3. A process as claimed in either claim 1 or claim 2 wherein the source of silver ions is silver oxide.

4. A process as claimed in any of claims 1—3 when performed at ambient temperature.

5. A process as claimed in any of claims 1—4 adapted to the preparation of methyl 9-O-methylclavulanate, ethyl 9-O-methylclavulanate, methoxymethyl 9-O-methylclavulanate or ethoxymethyl 9-O-methylclavulanate.

6. A process as claimed in any of claims 1—4 adapted to the preparation of benzyl 9-O-methylclavulanate.

7. A process as claimed in any of claims 1—6 wherein the molecular sieve is a sodium aluminium silicate.

8. A process as claimed in any of claims 1—6 in which calcium sulphate is used.

**Revendications**

1. Procédé pour la production d'acide 9-O-méthylclavulanique ou d'un sel ou ester hydrolysable in vivo pharmaceutiquement acceptable de celui-ci, ce procédé consistant à effectuer la réaction d'un

ester dissociable d'un acide clavulanique avec de l'iodure de méthyle ou du bromure de méthyle dans un solvant organique inerte anhydre en présence d'ions argent puis:

i) à convertir tout groupe ester dissociable qui n'est pas hydrolysable in vivo en un acide libre ou un sel ou groupe ester hydrolysable in vivo pharmaceutiquement acceptable;

ii) à convertir facultativement tout group ester dissociable qui est hydrolysable in vivo en un acide libre, en un sel pharmaceutiquement acceptable ou en un groupe ester hydrolysable in vivo différent; caractérisé en ce que le procédé est mis en oeuvre en présence de tamis moléculaire ou de sulfate de calcium.

2. Procédé suivant la revendication 1, caractérisé en ce que le solvant est du dichlorométhane.

3. Procédé suivant la revendication 1 ou 2, caractérisé en ce que la source d'ions argent est de l'oxyde d'argent.

4. Procédé suivant l'une quelconque des revendications 1 à 3, caractérisé en ce qu'il est mis en oeuvre à la température ambiante.

5. Procédé suivant l'un quelconque des revendications 1 à 4, adapté à la préparation de 9-O-méthyl-clavulanate de méthyle, de 9-O-méthyl-clavulanate d'éthyle, de 9-O-méthyl-clavulanate de méthoxyméthyle ou de 9-O-méthyl-clavulanate d'éthoxyméthyle.

6. Procédé suivant l'une quelconque des revendications 1 à 4, adapté à la préparation de 9-O-méthyl-clavulanate de benzyle.

7. Procédé suivant l'une quelconque des revendications 1 à 6, caractérisé en ce que le tamis moléculaire est de l'alumino-silicate de sodium.

8. Procédé suivant l'une quelconque des revendications 1 à 6, caractérisé en ce qu'on utilise du sulfate de calcium.

**Patentansprüche**

1. Ein Verfahren zur Herstellung von 9-O-Methylclavulansäure oder eines pharmakologisch verträglichen Salzes oder in vivo hydrolysierbaren Esters derselben, die Maßnahmen der Reaktion eines spaltbaren Esters der Clavulansäure mit Methyljodid oder Methylbromid in einem trockenen inerten organischen Lösungsmittel in Gegenwart von Silberionen, und anschließend:

i) die Umwandlung einer etwaigen spaltbaren Estergruppe, die nicht in vivo hydrolysierbar ist, in eine freie Säure, ein pharmakologisch vertägliches Salz oder eine in vivo hydrolysierbare Estergruppe;

ii) gegebenenfalls die Umwandlung einer etwaigen spaltbaren Estergruppe, die in vivo hydrolysierbar ist, in eine freie Säure, ein pharmakologisch verträgliches Salz oder eine andere in vivo hydrolysierbare Estergruppe umfassend; dadurch gekennzeichnet, daß es in Gegenwart eines Molekularsiebs oder von Calciumsulfat durchgeführt wird.

2. Ein Verfahren wie in Anspruch 1 beansprucht, in dem das Lösungsmittel Dichlormethan ist.

3. Ein Verfahren wie in Anspruch 1 oder Anspruch 2 beansprucht, in dem die Quelle von Silberionen Silberoxid ist.

4. Ein Verfahren wie in einem der Ansprüche 1 bis 3 beansprucht, wenn es bei Raumtemperatur durchgeführt wird.

5. Ein Verfahren, wie in einem der Ansprüche 1 bis 4 beansprucht, angepaßt zur Herstellung von 9-O-Methylclavulansäure-methylester, 9-O-Methylclavulansäure-äthylester, 9-O-Methylclavulansäure-methoxymethylester oder 9-O-Methylclavulansäure-äthoxymethylester.

6. Ein Verfahren wie in einem der Ansprüche 1 bis 4 beansprucht, angepaßt zur Herstellung von 9-O-Methylclavulansäure-benzylester.

7. Ein Verfahren wie in einem der Ansprüche 1 bis 6 beansprucht, wobei das Molekularsieb ein Natrium aluminiumsilikat ist.

8. Ein Verfahren wie in einem der Ansprüche 1 bis 6 beansprucht, in dem Calciumsulfat verwendet wird.